(19) Europäisches Patentamt
European Patent Office
Office européen des brevets

(11) Publication number: 0 321 185 A2

# (12) EUROPEAN PATENT APPLICATION

(21) Application number: 88311785.5

(51) Int. Cl.4: C07H 17/08 , A61K 31/70

(22) Date of filing: 13.12.88

The title of the invention has been amended (Guidelines for Examination in the EPO, A-III, 7.3).

(30) Priority: 16.12.87 GB 8729369

(43) Date of publication of application: 21.06.89 Bulletin 89/25

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Davies, John Sidney
Beecham Pharmaceuticals Brockham Park
Betchworth Surrey RH3 7AJ(GB)

(74) Representative: Dayneswood, Trevor et al
Beecham Pharmaceuticals Great Burgh Yew Tree Bottom Road
Epsom Surrey KT18 5XQ(GB)

## (54) Erythromycin derivatives.

(57) 9-N,11-O-methylene derivatives of erythromycin-9-imine (wherein the methylene group is optionally substituted) of the general formula IA or IB:

EP 0 321 185 A2

## CHEMICAL COMPOUNDS

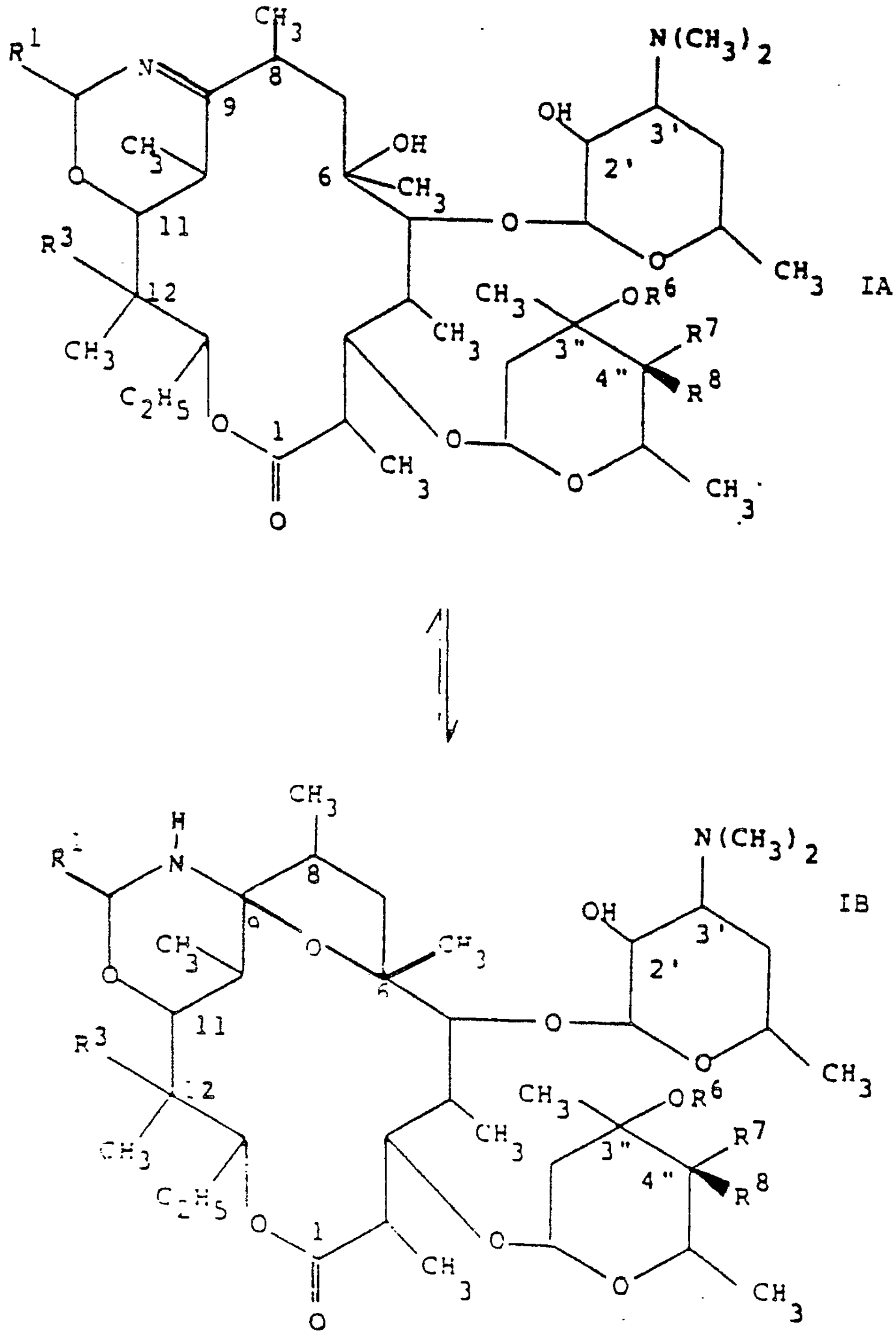

IA

IB

or a pharmaceutically acceptable ester or acid addition salt thereof,
wherein

$R^1$ denotes hydrogen, an unsubstituted or substituted hydrocarbon group, a heterocyclyl group, or an acyl group;

$R^3$ denotes hydrogen or hydroxy;

$R^6$ denotes hydrogen or methyl;

one of $R^7$ and $R^8$ denotes hydrogen, hydroxy, alkoxy,alkanoyloxy, amino, substituted amino, or a group of

## CHEMICAL COMPOUNDS

the formula $R^9$-$SO_2$-O-, and the other of $R^7$ and $R^8$ denotes hydrogen, or
$R^7$ and $R^8$ together denote an oxo group, an oxime group, or a substituted oxime group; and
$R^9$ denotes an organic group,
possess improved acid stability, compared with erythromycin A, whilst retaining good antibacterial activity.

The present invention relates to novel chemical compounds, their preparation and their use, and in particular to a novel class of erythromycin derivatives. These compounds have antibacterial properties, in particular against Gram-positive bacteria but also against some Gram-negative bacteria, and they are therefore of use in the treatment of bacterial infections in humans and animals caused by a wide range of organisms.

Erythromycin was first described in US 2 653 899 (R.L. Bunch et al; Eli Lilly). The structure of erythromycins can be represented as follows:

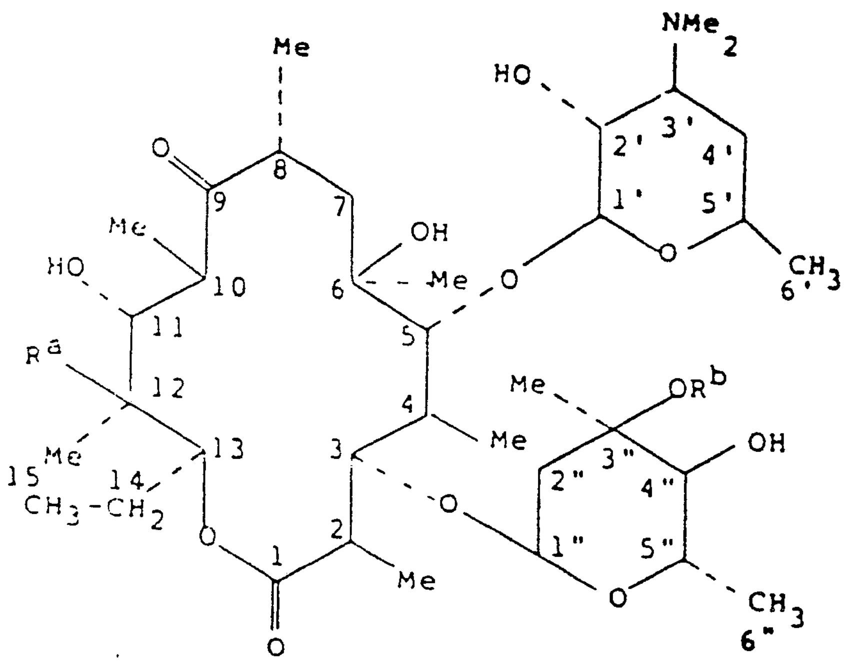

in which
$R^a$ denotes hydrogen or hydroxy and
$R^b$ denotes hydrogen or methyl.

The basic erythromycin structure comprises:

(i) a 14-membered lactone ring, referred to as the erythronolide ring, numbered with unprimed digits as shown in the above formula,

(ii) a first sugar ring, known as the desosamine ring, numbered with single-primed digits, and

(iii) a second sugar ring, known as the cladinose ring, numbered with double-primed digits.

The erythronolide ring can have two different substituents, $R^a$, at C-12:
erythronolide A (in which $R^a$ = OH)
erythronolide B (in which $R^a$ = H).

The four main naturally occurring erythromycins are as follows:

| Erythromycin | $R^a$ | $R^b$ |
|---|---|---|
| A | OH | $CH_3$ |
| B | H | $CH_3$ |
| C | OH | H |
| D | H | H |

of which erythromycin A is by far the most important.

Erythromycins, and in particular erythromycin A, are antibiotics widely employed clinically in the treatment of infections caused by Gram-positive and some Gram-negative bacteria. A major drawback of erythromycins is their poor acid stability, resulting in poor and erratic oral absorption.

Numerous attempts have been made to modify erythromycin to produce derivatives having improved acid stability without loss of the antibacterial activity.

(9S)-9-Dihydroerythromycin A (which carries a 9-hydroxy group in place of the 9-keto group) has been described, but has poor antibacterial activity (P.F. Wiley et al, J. Amer. Che. Soc., 1955, 77, 3676-3677; M.V. Signal et al, ibid, 1956, 78, 388-395; and T. Glabski et al, Roczniki Chem., 1976, 50, 1281). Erythromycylamine and erythromycin oxime (in which the 9-keto group is replaced, respectively, by an amino or oxime group), as well as various N-substituted derivatives of erythromycylamine have also been described (GB 1 100 504 (Pliva Pharmaceutical); E.H. Massey et al, Tetrahedron Letters, 1970, No. 2, 157-160; and G.H. Timms et al, ibid, 1971, No. 2 195-198), as have various erythromycin oxime ethers (US 3 681 326 (A.M. Von Esch; Abbott Laboratories); US 3 869 445 and US 4 063 014 (both R. Hallas et al; Abbott Laboratories); US 4 349 545 (S. Gouin d'Ambrieres; Roussel-Uclaf)); and S. Pestka et al, Antimicrobial agents and chemotherapy, 1974, 6 479).

Erythromycin 9-imine has also been described, as an intermediate in the preparation of erythromycylamine (G.H. Timms et al, op. cit). 11-ether and 6-carbamate derivates of erythromycin 9-imine have been disclosed in EP 0 201 166 and EP 0 216 169 respectively (both Beecham).

Certain aldehyde-erythromycylamine condensation products with a 9-N,6-O- or 9-N,11-O-cyclic substituent have previously been disclosed (US 4 048 306 (R. Maier et al; Boehringer Ingelheim GmbH)) and EP 0 238 178 (Bonjouklian et al, Eli Lilly + Co.)).

4″-Deoxy-11-O-methylthiomethyl-4″-oxo-erythromycin B and its conversion to (i) 4″-deoxy-9,11-O-(optionally substituted)methylene-4″-oxo-erythromycin B 6,9-hemiacetal and the corresponding 4″-epi-hydroxy, 2′,4″-O-diacetyl-4″-epi, and 4″-O-acetyl-4″-epi derivatives, and (ii) 4″-deoxy-4″-oxo-, 4″-O-acetyl-4″-epi-, and 4″-epi-erythromycin B; as well as 4″-O-formyl-11-O-methylthiomethyl-erythromycin B and its conversion to 11-O-methylthiomethyl-erythromycin B, 9,11-O-methylene-erythromycin B 6,9-hemiacetal, 11-O-methyl-erythromycin B and 11-O-n-butyl-erythromycin B; and also 4″-deoxy-4″-oxo-erythromycin A are described in US 3 842 069, US 3 884 903 and US 3 884 904 (all P.H. Jones et al; Abbott Laboratories).

4″-Deoxy-4″-amino-erythromycin A, 4″-deoxy-4″-amino-erythromycin A 6,9-hemiketal, and 4″-deoxy-4″-oxo-erythromycin A 6,9-hemiketal, as well as corresponding 11-O-acetyl and 11,12-cyclic carbonate derivates, and also 4″-deoxy-4″-amino-erythromycin B and 4″-deoxy-4″-oxo-erythromycin A 4″-O-oxime or 4′-O-acetyloxime, are described in US 4 150 220 (F.C. Sciavolino; Pfizer).

Various 4″-O-sulphonyl derivatives of erythromycin, erythromycin oxime, and erythromycin oxime ethers are described in US 3 836 519, US 3 869 445 and US 4 063 114 (all R. Hallas et al; Abbott Laboratories). Certain further 4″-deoxy-erythromycin derivatives are described in JP 58-049396 (Toyo Jozo KK).

An 11,12-cyclic carbonate of 9-dihydroerythromycin has also been described in T. Glabski et al; Roczniki Chem., 1976, 50, 1281 and 9-dihydro-11,12-O-isopropylidene-erythromycin A and the corresponding 4″-epi compound, as well as 4″-epi-erythromycin A and corresponding 9-dihydro, 11,12-carbonate, 9-dihydro-11,12-carbonate, and 2′-acetyl compounds, have been described in US 4 382 085 and US 4 382 086 (both F.C. Sciavolino et al; Pfizer).

11,12-O-(optionally substituted)-methylene derivatives and 9,11-O-(optionally substituted)-methylene derivatives of erythromycin are described in WO 86/01513 and EP 0 184 921 A2, respectively (both Beecham).

6-O-Methyl-, 6,11-di-O-methyl-, 11-O-methyl- and 11-O-ethyl-erythromycin A, and also 6-O-methyl-6,4″-di-O-methyl-, and 6,11,4″-tri-O-methyl-erythromycin B, as well as 6-O-methyl-erythromycin A 9-oxime, are described in EP 0 041 355 A1, EP 0 080 818 A1, EP 0 080 819 A1, EP 0 158 467 A2, and EP 0 180 415 A2, (all Taisho Pharmaceutical).

8-hydroxy-erythromycin A and 8-fluoro-erythromycin A have been described (K. Krowicki et al, J.Antibiotics, XXVI 575-581 (1973), and L.Toscano et al, ibid XXXVI 1439-1450 (1983), respectively). Various

6,9-ketal derivatives of 8-fluoro-erythromycins have also been described (EP 0 158 102 A, Pierrel).

The erythromycin derivatives according to the present invention possess improved acid stability as compared with erythromycin A while retaining good antibacterial activity.

The present invention provides antibacterially active 9-N,11-O-methylene derivatives of erythromycin 9-imine wherein the methylene group is optionally substituted.

In particular, the present invention provides a compound of the general formula IA or IB or a pharmaceutically acceptable ester or acid addition salt thereof:

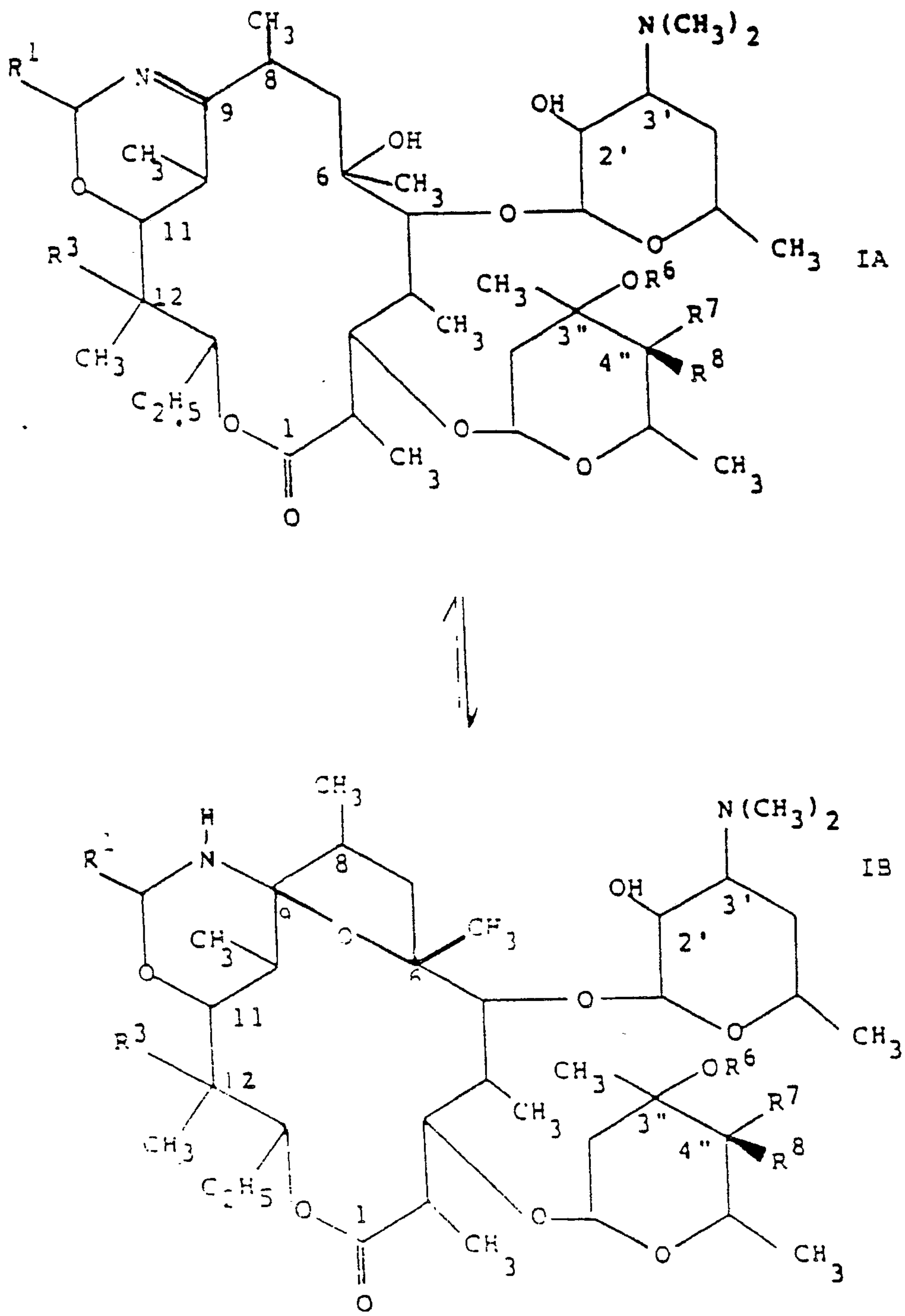

IA

IB

wherein

$R^1$ denotes hydrogen, an unsubstituted or substituted hydrocarbon group, a heterocyclyl group, or an acyl group;

$R^3$ denotes hydrogen or hydroxy;

$R^5$ denotes hydrogen or methyl;
one of $R^7$ and $R^8$ denotes hydrogen, hydroxy, alkoxy, alkanoyloxy, amino, substituted amino, or a group of the formula $R^9$-$SO_2$-O-, and the other of $R^7$ and $R^8$ denotes hydrogen, or
$R^7$ and $R^8$ together denote an oxo group, an oxime group, or a substituted oxime group; and
$R^9$ denotes an organic group.

The compounds according to the present invention may exist in the form shown in formula IA or formula IB, the two forms being in equilibrium. References hereinafter to "formula I" encompass both forms unless the context requires otherwise.

The term 'hydrocarbon' as used herein includes groups having up to 18 carbon atoms, suitably up to 10 carbon atoms, conveniently up to 6 carbon atoms. Suitable hydrocarbon groups include $(C_{1-6})$alkyl, $(C_{2-6})$-alkenyl, $(C_{2-6})$alkynyl, $(C_{3-7})$cycloalkyl, aryl, $(C_{3-7})$cycloalkyl$(C_{1-6})$alkyl, aryl$(C_{1-6})$alkyl, $(C_{1-6})$alkyl$(C_{3-7})$-cycloalkyl, and $(C_{1-6})$alkylaryl.

Examples of suitable optional substituents for the above mentioned hydrocarbon groups include, heterocylyl, amino, $(C_{1-6})$alkanoylamino, (mono, di, or tri)-$(C_{1-6})$alkylamino, hydroxy, $(C_{1-6})$alkoxy, $(C_{1-6})$-alkoxy$(C_{1-6})$alkoxy, aryloxy, mercapto, $(C_{1-6})$alkylthio, heterocyclylthio, arylthio, sulphamoyl, carbamoyl, substituted carbamoyl, amidino, guanidino, nitro, chloro, bromo, fluoro, carboxy and salts and esters thereof, $(C_{1-6})$alkanoyloxy, arylcarbonyloxy, heterocyclylcarbonyloxy, and acyl groups.

Any alkyl group or moiety referred to herein may be straight or branched, unsubstituted or substituted, and may contain, for example, up to 12 carbon atoms, suitably up to 6 carbon atoms. In particular, the alkyl group or moiety may be an unsubstituted or substituted methyl, ethyl, n-propyl, iso-propyl, n-butyl, sec-butyl, isobutyl or tert-butyl group. Examples of suitable optional substitutents for any such alkyl group or moiety include the above-listed substitutents for hydrocarbon groups, and also the above-listed non-alkyl hydrocarbon groups, for example $(C_{2-6})$alkenyl and aryl groups.

The term 'aryl' as used herein includes phenyl and naphthyl, which may be unsubstituted or substituted by up to five, preferably up to three, groups selected from the above-listed substituents for hydrocarbon groups, and the above-listed hydrocarbon groups, including, for example substituents selected from halogen, $(C_{1-6})$alkyl, phenyl, $(C_{1-6})$alkoxy, halo$(C_{1-6})$alkyl, hydroxy, amino, nitro, carboxy, $(C_{1-6})$-alkoxycarbonyl, $(C_{1-6})$alkoxycarbonyl$(C_{1-6})$alkyl, $(C_{1-6})$alkanoyloxy, and $(C_{1-6})$alkanoyl groups.

The term 'acyl' as used herein includes formyl, unsubstituted and substituted hydrocarbon-carbonyl and hydrocarbonoxy-carbonyl groups, including, for example, unsubstituted and substituted alkanoyl, cycloalkylcarbonyl, arylcarbonyl, alkoxycarbonyl, aryloxycarbonyl, and heterocyclylcarbonyl groups. The term 'acyloxy' is used analogously.

The terms 'heterocyclyl' and 'heterocyclic' as used herein include aromatic and non-aromatic, single and fused, rings suitably containing up to four hetero-atoms in each ring selected from oxygen, nitrogen and sulphur, which rings may be unsubstituted or substituted by, for example, up to three groups selected form halogen, $(C_{1-6})$alkyl, $(C_{1-6})$alkoxy, halo$(C_{1-6})$alkyl, hydroxy, amino, carboxy, carboxy salts, carboxy esters, $(C_{1-6})$alkoxycarbonyl, $(C_{1-6})$alkoxycarbonyl$(C_{1-6})$alkyl, aryl, and oxo groups. Each heterocyclic ring suitably has from 4 to 7, preferably 5 or 6, ring atoms. A fused heterocyclic ring system may include carbocyclic rings and need include only one heterocyclic ring.

The term 'heteroaryl' as used herein means an aromatic heterocyclic ring or ring system, suitably having 5 or 6 ring atoms in each ring.

The compounds according to the present invention, of the general formula I, are 9-N,11-O-methylene derivatives of erythromycin 9-imine.

A hydrocarbon group denoted by $R^1$ may suitably be an unsubstituted or substituted alkyl, cycloalkyl, or aryl group. Advantageously, the alkyl group may be a lower alkyl group, for example a $(C_{1-6})$alkyl group. Advantageously, the cycloalkyl group may be a cyclohexyl group. Advantageously, the aryl group may be a phenyl group.

A heterocyclyl group denoted by $R^1$ may suitably be a furyl, thienyl, dihydrofuryl, tetrahydrofuryl, dihydrothienyl, tetrahydrothienyl, pyranyl, dihydropyranyl, tetrahydropyranyl, thiapyranyl, dihydrothiapyranyl, tetrahydrothiapyranyl, pyrrolyl, dihydropyrrolyl, tetrahydropyrrolyl, pyridyl, dihydropyridyl, tetrahydropyridyl, hexahydropyridyl or triazolyl ring.

An acyl group denoted by $R^1$ may suitably be an unsubstituted or substituted alkanoyl group, for example a $(C_{1-6})$alkanoyl group.

In preferred compounds of the general formula I, $R^1$ denotes hydrogen, unsubstituted or substituted alkyl (especially lower alkyl), unsubstituted or substituted aryl (especially phenyl), unsubstituted or substituted heterocyclyl (especially furyl, thienyl or triazolyl), or acyl (especially unsubstituted or substituted alkanoyl).

Examples of suitable substitutents for a hydrocarbon or heterocyclyl group $R^1$, and for inclusion in an

acyl group (for example, a substituted alkanoyl group) $R^1$, include, in particular, alkyl, alkoxy, aryl, aryloxy, hydroxy, halo (for example, chloro, bromo or iodo), azido, nitro, amino, substituted amino (for example, monoalkylamino, dialkylamino and alkanoylamino), carboxy, esterified carboxy (for example, alkoxycarbonyl), acyloxy (for example, alkanoyloxy), carbamoyl ($H_2N$-C(=O)-), and substituted carbamoyl (for example, N-alkylcarbamoyl and N,N-dialkylcarbamoyl) groups. Any alkyl or aryl moiety in such substituents may itself be substituted by, for example, one of the listed substituents, and any alkyl moiety advantageously contains not more than 6, preferably not more than 4, carbon atoms. An example of a substituent in which an alkyl or aryl moiety is itself substituted is an alkoxyalkoxy or alkylaryl substituent.

In the compounds of the general formula I, preferably $R^3$ denotes a hydroxy group as in the erythronolide A ring or, in other words, the compounds of the general formula I are preferably derivatives of erythromycin A 9-imine. Alternatively, however, the present compounds may be derivatives of erythromycin B 9-imine, in which case $R^3$ denotes a hydrogen atom, or of the 9-imine of another naturally occurring erythromycin.

The -$OR^6$ group in the 3″-position of the cladinose ring may be a hydroxy group or a methoxy group. Preferably, $R^6$ denotes a methyl group as in erythromycin A and B.

The 4″-position of the cladinose ring may suitably carry a hydroxy group as in erythromycin A and B ($R^7$ = H, $R^8$ = OH). Various modifications of the 4″-position of the cladinose ring have previously been described and those modifications may be incorporated in the compounds according to the present invention:

(i) 4″-deoxy-4″-oxo derivatives ($R^7$ + $R^8$ = O=) are described in US 3 842 069, US 3 884 903 and US 4 150 220, all op. cit.;

(ii) 4″-epi-hydroxy derivatives ($R^7$ = OH; $R^8$ = H) and 4″-deoxy-4″-alkanoyloxy-4″-epi derivatives ($R^7$ = alkanoyloxy, especially $CH_3COO$-; $R^8$ = H) are described in US 3 884 903 and US 4 382 085, both op. cit.;

(iii) 4″-O-alkyl derivatives ($R^7$ or $R^8$ = alkoxy, especially methoxy; the other of $R^7$ and $R^8$ = H) are described in EP 0 080 818 A1, op. cit.;

(iv) 4″-deoxy-4″-amino derivatives ($R^7$ or $R^8$ = amino or substituted amino; the other of $R^7$ and $R^8$ = H) are described in US 4 150 220, op. cit.;

(v) 4″-deoxy-4″-oxime derivatives ($R^7$ + $R^8$ = oxime (=N-OH) or substituted oxime, especially acetyloxime (=N-O-CO-$CH_3$)) are also described in US 4 150 220, op cit.;

(vi) 4″-O-sulphonyl derivatives ($R^7$ = H, $R^8$ = $R^9$-$SO_2$-O-) are described in US 3 836 519, US 3 869 445 and US 4 063 014, all op. cit.; and

(vii) 4″-deoxy derivatives ($R^7$ = $R^8$ = H) are described in JP 58-049396, op. cit..

In the 4″-deoxy-4″-(substituted amino) derivatives, the substituted amino group $R^7$ or $R^8$ may suitably be a group of the formula

-$NHCOR^C$ or -$NHSO_2R^C$

in which $R^C$ denotes a hydrocarbon group.

In the 4″-O-sulphonyl derivatives, in which $R^7$ or $R^8$ denotes a sulphonyloxy group of the formula

$R^9$-$SO_2$-O-,

the organic group $R^9$ may suitably be an unsubstituted or substituted hydrocarbon or oxahydrocarbon group, thiahydrocarbon or azahydrocarbon group, more especially an alkyl, alkenyl, unsubstituted or substituted aryl (especially phenyl, nitrophenyl, halophenyl or alkylphenyl), unsubstituted or substituted aralkyl (especially benzyl, nitrobenzyl, halobenzyl or alkylbenzyl), unsubstituted or substituted aryloxyalkyl (especially phenoxyalkyl, nitrophenoxyalkyl, halophenoxyalkyl or alkylphenoxyalkyl), or substituted ethyl (especially $R^D$-$CH_2$-$CH_2$-, wherein $R^D$ is defined as below) group.

Examples of groups $R^D$ in the 4″-substituent

$R^D$-$CH_2$-$CH_2$-$SO_2$-O-

include amino, substituted amino, carbamoyl, substituted carbamoyl, sulphamoyl, substituted sulphamoyl, substituted ureido, substituted thioureido, alkoxy, alkythio, optionally substituted aryloxy, optionally substituted arylthio, optionally substituted benzyloxy, optionally substituted benzylthio, substituted sulphonyl, substituted sulphinyl, substituted alkyl, substituted alkanoyl, substituted cyano, and other groups more specifically described in US 3 869 445 and US 4 063 014, op. cit..

Preferably, $R^9$ denotes a hydrocarbon group, particularly a ($C_{1-6}$)alkyl group, especially a methyl group.

The present invention includes pharmaceutically acceptable esters, especially in vivo hydrolysable esters, of the compounds of the general formula I. Such esters may be formed at any hydroxy group in the compounds of the general formula I, but usually the ester will be formed at the 2′-hydroxy group of the

desosamine ring, thus giving a 2′-O-acyl derivative of the type described in US 2 862 921 (R.E. Booth et al; Upjohn Co.), US 2 993 833 (V.C. Stephens; Eli Lilly), US 3 836 519, US 3 842 069, US 3 869 445, US 3 884 903, US 3 884 904 and US 4 150 220, all op. cit..

Suitable pharmaceutically acceptable in vivo hydrolysable esters include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic, and alkanedioic acids, in which each alkyl or alkenyl moiety advantageously has not more than 6 carbon atoms. Examples of particular esters include formates, acetates, propionates, butyrates, acrylates, and ethylsuccinates.

The present invention also includes acid addition salts, especially pharmaceutically acceptable acid addition salts, of the compounds of the general formula I. Such acid addition salts may, in particular be formed at the 3′-dimethylamino group of the desosamine ring.

Various acid addition salts of erythromycin are described in US 2 761 859 (C.E. Hoffhine, Jr.; Abbott Laboratories) and US 2 852 429 (J.T. Shepler; Eli Lilly).

Suitable acid addition salts of the compounds of the invention include pharmaceutically acceptable inorganic acid addition salts, for example the sulphate, nitrate, phosphate, borate, hydrochloride and hydrobromide, and also pharmaceutically acceptable organic acid addition salts, for example the acetate, tartrate, maleate, citrate, succinate, benzoate, ascorbate, methanesulphonate, α-keto-glutarate, α-glycerophosphate, and glucose-1-phosphate. Preferably the acid addition salt is the laurylsulphate salt.

Examples of individual compounds according to the prevent invention include:

(i) 9,11-N,O-benzylideneerythromycin A 9-imine
(ii) 9,11-N,O-methyleneerythromycin A 9-imine
(iii) 9,11-N,O-ethylideneerythromycin A 9-imine
(iv) 9,11-N,O-(2-thienylmethylene)erythromycin A 9-imine
(v) 9,11,N-O-cyclohexylmethyleneerythromycin A 9-imine
(vi) 9,11-N,O-propylideneerythromycin A 9-imine
(vii) 9,11-N,O-p-methylbenzylideneerythromycin A 9-imine
(viii) 9,11-N,O-furfurylideneerythromycin A 9-imine
(ix) 9,11-N,O-p-chlorobenzylideneerythromycin A 9-imine
(x) 9,11-N,O-(2-oxapropylidene)erythromycin A 9-imine
(xi) 9,11-N,O-(3-ethoxypropylidene)erythromycin A 9-imine
(xii) 9,11-N,O-p-nitrobenzylideneerythromycin A 9-imine
(xiii) 9,11-N,O-p-methoxybenzylideneerythromycin A 9-imine
(xiv) 9,11-N,O-butylideneerythromycin A 9-imine
(xv) 9,11-N,O-pentylideneerythromycin A 9-imine
(xvi) 9,11-N,O-(2,2,2-trimethylethylidene)erythromycin A 9-imine
(xvii) 9,11-N,O-(2-phenylethylidene)erythromycin A 9-imine
(xviii) 9,11-N,O-p-fluorobenzylideneerthyromycin A 9-imine
(xix) 9,11-N,O-p-acetamidobenzylideneerythromycin A 9-imine
(xx) 9,11-N,O-(3-phenylprop-2-enylidene)erythromycin A 9-imine
(xxi) 9,11-N,O-(pyridine-4-yl)methyleneerythromycin A 9-imine
(xxii) 9,11-N,O-(1-methyl-1,2,3-triazol-4-yl) methyleneerythromycin A 9-imine
(xxiii) 9,11-N,O-(2-hydroxyethylidene)erythromycin A 9-imine
(xxiv) 9,11-N,O-(2-azidoethylidene)erythromycin A 9-imine
(xxv) 9,11-N,O-(2-aminoethylidene)erythromycin A 9-imine
(xxvi) 9,11-N,O-(2-N,N-dimethylethylidene)erythromycin A 9-imine

as well as
the corresponding isomers in the form of formula IB, and
the corresponding derivatives in which the 4″-position is modified as discussed above;
and also
pharmaceutically acceptable esters and acid addition salts of such compounds.

The 9-N,11-O-methylene erythromycin 9-imine derivatives according to the invention may be prepared by reacting an erythromycin 9-imine or derivative thereof, having a hydroxy substituent at the 11-position, in which any reactive groups (other than the 9-imino and 11-hydroxy groups) may optionally be protected, with an aldehyde or a reactive derivative thereof; and thereafter, if necessary, carrying out one or more of the following steps:

(a) converting a substitutent on the erythromycin structure to another such substituent in a conventional manner;

(b) removing any protecting groups; and

(c) forming a pharmaceutically acceptable ester or acid addition salt.

More particularly, a compound of the general formula I as hereinbefore defined or a pharmaceutically acceptable ester or acid addition salt thereof may be prepared by a process which comprises reacting a compound of the general formula IIIA or IIIB:

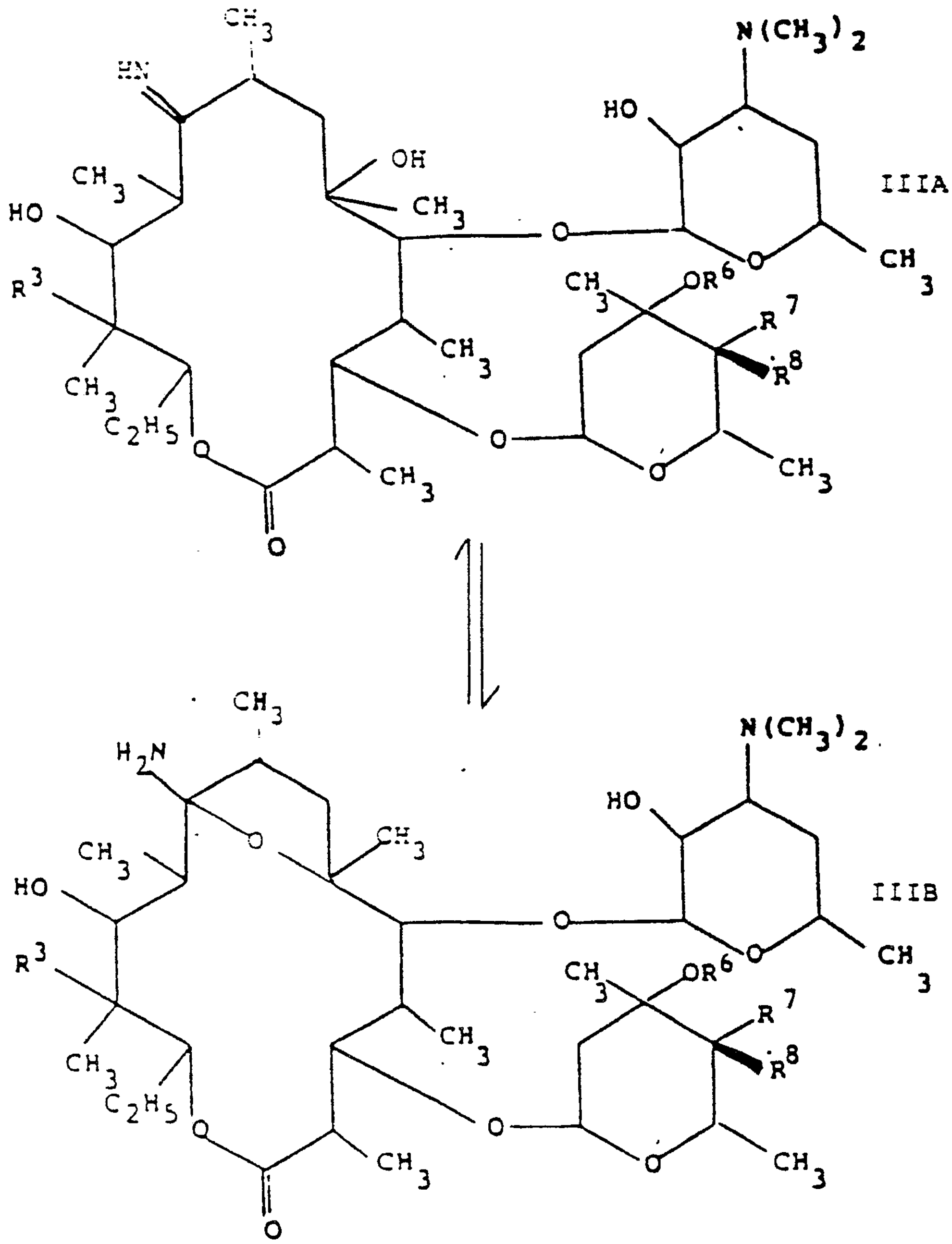

IIIA

IIIB

wherein $R^3$, $R^6$, $R^7$ and $R^8$ are defined as above with respect to general formula I, in which compound of the general formula III any reactive group (other than the 9-imino and 11-hydroxy groups) may optionally be protected, with:

a compound of the general formula IV:

$R^1$-CH=O IV

in which $R^1$ is defined as above with respect to general formula I, or a reactive derivative of such a compound;

and thereafter removing any protecting group that may be present;

and optionally

(a) converting either or both of groups $R^7$ and $R^8$ to another such group; and or
(b) forming a pharmaceutically acceptable ester or acid addition salt.

Analogously to the compounds of the formula I, the compounds of the formula III may exist in the form shown in formula IIIA or formula IIIB, the two forms being in equilibrium. References herein to 'formula III' encompass both forms unless the context requires otherwise.

The compound of the general formula III in which:
each of $R^3$ and $R^8$ denotes hydroxy,
$R^7$ denotes hydrogen, and
$R^5$ denotes methyl
is erythromycin A 9-imine, which may, for example, be prepared by the reduction of erythromycin A 9-oxime with trivalent titanium (for example, titanium trichloride) as described by G.H. Timms et al, op. cit..

The compound of the general formula III in which:
each of $R^3$ and $R^7$ denotes hydrogen,
$R^5$ denotes methyl, and
$R^8$ denotes hydroxy
is erythromycin B 9-imine, and may, for example, be prepared by reduction of erythromycin B 9-oxime by analogous methods.

Other compounds of the general formula III may also be prepared, by methods known per se, from erythromycin A or B or the corresponding 9-oxime derivatives. For example, a compound in which the 4″-position is substituted other than as in naturally-occuring erythromycin A or B (that is to say, in which $R^7$ is other than hydrogen and/or $R^8$ is other than hydroxy) may be prepared as described in the respective references cited above.

In general, in the preparation of compounds of the general formula III, the formation of the 9-imine group will be effected subsequent to modification of other positions of the erythromycin molecule.

Prior to carrying out the reaction of a compound of the general formula III with a compound of the general formula IV or a reactive derivative thereof, any reactive group of a compound of the general formula III may optionally be protected. It has been found, however, that such protection is often not necessary when carrying out the process of the present invention, except in cases where more severe reaction conditions are required.

When protection is desired, the 3′-dimethylamino group may be protected by an N-protecting group. The N-protection may be effected in known manner, for example by the method described by E.H. Flynn et al, (J. Amer. Chem. Soc.,1955, 77, 3104-3106).

Examples of suitable N-protecting groups include benzyloxycarbonyl, and substituted benzyloxycarbonyl, (for example, p-methylbenzyloxycarbonyl, p-nitrobenzyloxycarbonyl, p-bromobenzyloxycarbonyl, p-phenylazobenzyloxycarbonyl, and p-(p′-methoxyphenylazo)-benzyloxycarbonyl). A preferred N-protecting group is benzyloxycarbonyl.

Additionally, one or more of the hydroxy groups present in the erythromycin molecule (other than the 11-hydroxy group) may be protected prior to the reaction. In particular, any hydroxy groups present at the 2′- and 4″-positions, especially the 2′-hydroxy group, may be protected. It is convenient to employ the same group to protect the hydroxy group(s) as that employed to protect the amino moiety, especially a benzyloxycarbonyl group.

Any reactive substituents that may be present in the group $R^7$ or $R^8$ may also be protected in a conventional manner.

It has been found that, when N-protection of the 3′-dimethylamino group is desired, it can be particularly advantageous to effect such N-protection prior to formation of the 9-imine compound of the general formula III. Although the 9-imine compounds of the general formula III are in general sufficiently stable to be isolated and subjected to various further reactions (including the process according to the invention), at least some such compounds are nevertheless not very stable, as demonstrated by the fact that, on subjecting erythromycin A 9-imine to silica gel chromatography, it may convert to erythromycin A. On the other hand, it has now been found that the corresponding 3′-N-protected 9-imine compounds, e.g. 3′-N-desmethyl-2′-O,3′-N-bisbenzyloxycarbonyl-erythromycin A 9-imine, can be obtained from the corresponding 3′-N-protected 9-oxime compounds (for example, by reduction with trivalent titanium as described above), and that such 3′-N-protected 9-imine compounds are surprisingly considerably more stable than the corresponding unprotected 9-imine compounds, as demonstrated by the fact that the aforementioned 3′-N-protected erythromycin A 9-imine can readily be chromatographed on silica gel without decomposition.

The 3′-N-protected erythromycin 9-imines are novel compounds and accordingly the present invention also provides compounds of the general formula VA or VB, which may be used as protected forms of the compounds of the general formula III in the process according to the invention:

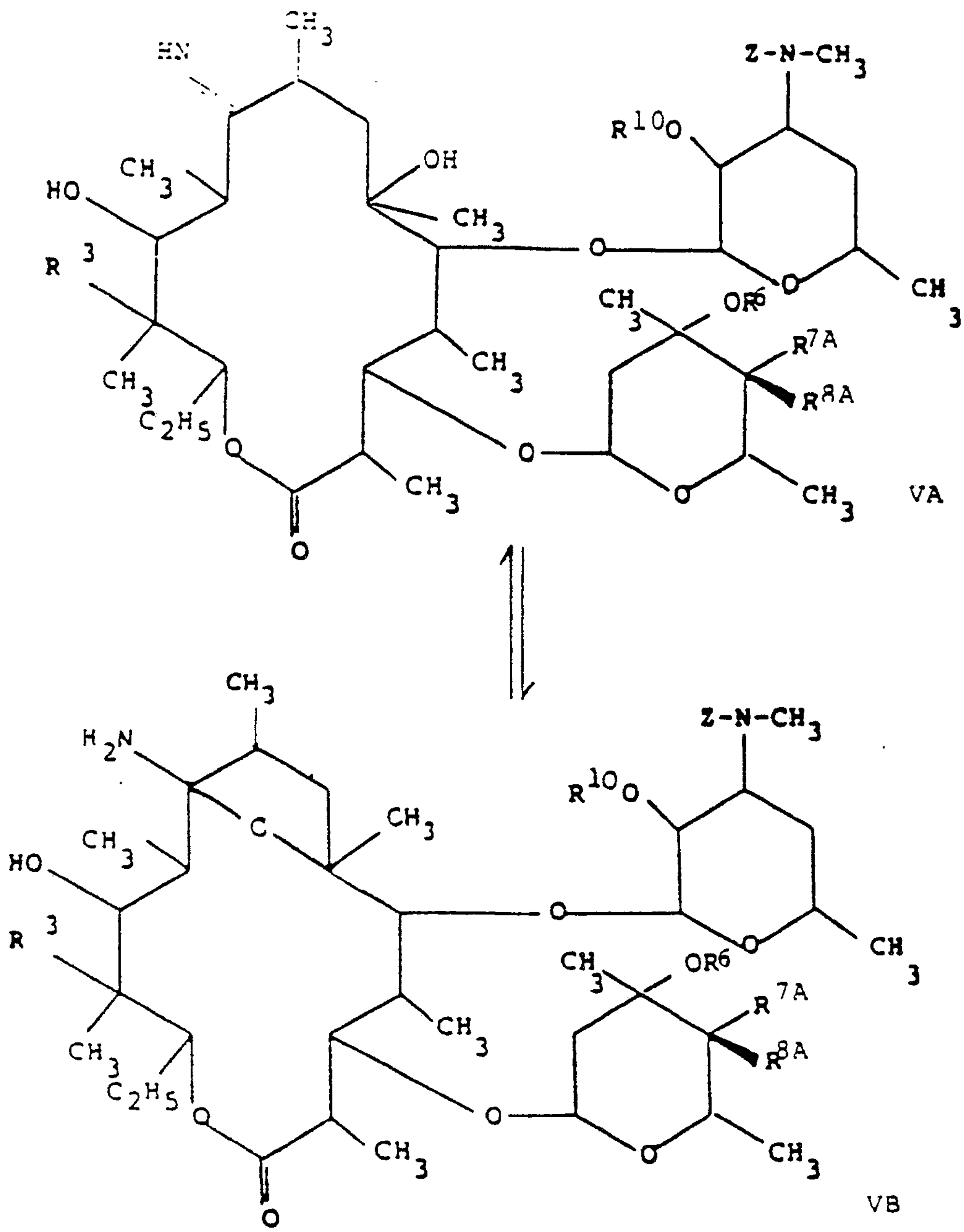

VA

VB

in which

$R^3$ and $R^6$ are defined as above with respect to general formula I;

one of $R^{7A}$ and $R^{8A}$ denotes H, OH, OZ, $NZ_2$, $NH_2$, NHZ, substituted $NH_2$, substituted NHZ, alkanoyloxy, or $R^9$-$SO_2$-O- (in which $R^9$ denotes an organic group) and the other of $R^{7A}$ and $R^{8A}$ denotes H, or

$R^{7A}$ and $R^{8A}$ together denote oxo;

$R^{10}$ denotes H or Z; and

Z denotes a protecting group, more particularly an N-protecting group, preferably a substituted benzyloxycarbonyl group or, especially, a benzyloxycarbonyl group.

Analogously to the compounds of the formula III, the compounds of the formula V may exist in the form shown in formula VA or formula VB, the two forms being in equilibrium. References herein to 'formula V' encompass both forms unless the context requires otherwise.

In the process according to the invention, the compound of the general formula III, optionally containing

protective groups, is reacted with an aldehyde of the general formula IV or a reactive derivative thereof. Suitable reactive derivatives of aldehydes of the general formula IV include, for example, acetals of the general formula VI

$$\begin{array}{c} R^{1} \\ | \\ R^{11}O\text{-}CH\text{-}OR^{12} \end{array} \qquad VI;$$

and hemiacetals of the general formula VII

$$\begin{array}{c} R^{1} \\ | \\ R^{11}O\text{-}CH\text{-}OH \end{array} \qquad VII;$$

in which formula VI and VII.

$R^1$ is defined as above with respect to general formula I; and each of $R^{11}$ and $R^{12}$, which may be identical or different, denotes a hydrocarbon group, advantageously a $(C_{1-6})$hydrocarbon group, preferably an alkyl group, especially a methyl or ethyl group.

When the process according to the invention is being carried out using a reactive derivative of the aldehyde of the formula IV, that is to say, for example, using a compound of one of the formulae Vi or VII above, the erythromycin 9-imine is suitably used in protected form, in particular with the 3′-dimethylamino group protected as discussed above.

When using a reactive derivative of the aldehyde, the process is also suitably carried out in the presence of an acid catalyst. Preferred acid catalysts include pyridinium salts, for example pyridinium tosylate and pyridinium chloride. Other suitable acid catalysts include, for example, zinc chloride, cupric sulphate, boron trifluoride etherate, organic sulphonic acids (for example, p-toluenesulphonic acid), and acid ion exchangers, all optionally in conjunction with, for example, tertiary organic bases (for example, pyridine, dimethylpyridines, and trimethylpyridines).

Advantageously, the reaction is also carried out in the presence of a drying agent, for example anhydrous calcium sulphate, magnesium sulphate, sodium sulphate, cupric sulphate, or molecular sieves.

When using an unprotected erythromycin 9-imine and an aldehyde of the general formula IV, the process according to the invention may suitably be carried out in the presence of a hydroxylic solvent, preferably an alcohol, especially an alkanol, for example ethanol.

When using a protected erythromycin derivative of the general formula III and/or a reactive derivative of an aldehyde (such as a compound of the general formula VI or VII), the process according to the invention may suitably be carried out in an inert solvent. Suitable solvents include, for example, ether solvents (for example, tetrahydrofuran, dioxan, ethoxyethane, and 1,2-dimethoxyethane), halogenated solvents (for example, chloroform and methylene chloride), and aromatic solvents (for example, toluene).

The process according to the invention may suitably be effected at a cool to slightly elevated temperature, preferably at ambient temperature. The reaction may, for example, be effected at a temperature within the range of from -30° C to +30° C, preferably from 0° C to +30° C, especially from +10° C to +25° C.

After completion of the reaction with the aldehyde or reactive derivative thereof, and suitably prior to removal of any protecting groups, either of the groups $R^7$ and $R^8$ may be converted to any of the other such groups within the definitions given above by methods known in the art, for example by the methods disclosed in the publications mentioned above. For example, a compound in which R8 denotes hydrogen and $R^7$ denotes hydroxy can be converted to a compound in which $R^7$ and $R^8$ together denote oxo and optionally thereafter to a compound in which $R^8$ denotes hydroxy or acetoxy and $R^7$ denotes hydrogen by methods analogous to those described in US 3 884 903, op.cit..

After completion of the reaction with the aldehyde or reactive derivative thereof and after the optional conversion of any groups $R^7$ and $R^8$, any protecting groups may be removed by a conventional method. It is preferable to employ a hydrogenation procedure.

The hydrogenation may suitably be carried out in the presence of a transition metal catalyst, for example palladium, which may, for example, be in the form of palladium on carbon (charcoal), palladium on barium sulphate, palladium on calcium carbonate, or palladium black. A favoured catalyst is palladium on

carbon (sometimes referred to as palladium on charcoal); for example 5%, 10%, 20% or 30% palladium on carbon. A low, medium or high pressure of hydrogen may be used in this reaction, for example a pressure of from 1 to 6 atmospheres absolute, a pressur of 1 atmosphere absolute being convenient. The reaction may suitably be carried out at a non-extreme temperature, for example at a temperature within the range of from 0° C to 30° C, preferably from 12° C to 25° C. It is generally convenient to carry out the reaction at ambient temperature. The reaction is preferably carried out at a pH within the range of from 4.5 to 5.0, which may be maintained by the use of a suitable buffer, for example an acetate buffer at pH 4.8. Suitable solvents for carrying out the hydrogenation include ethanol, n-propanol, isopropanol, tetrahydrofuran, dioxan, ethyl acetate, a mixture of two or more such solvents, or such a solvent or mixture in the presence of water. A favoured solvent is ethanol.

In order to restore the dimethylamino group at the 3′-position, it is convenient to effect a reductive methylation, which advantageously may be carried out at the same time as the reductive removal of the protecting groups, as in the method of Flynn et al, op.cit..

The reductive methylation method commonly used involves the use of formaldehyde. Thus, it is possible to effect the reaction of a protected starting material of the general formula V with formaldehyde (as the aldehyde of the formula IV), simultaneously with the hydrogenation and reductive methylation for removal of the protecting group(s) Z. For example, 3′-N-desmethyl-2′-O-3′-N-bisbenzyloxycarbonyl-erythromycin A 9-imine may be subjected to simultaneous hydrogenation and reductive methylation using formaldehyde, according to the method of Flynn et al, op. cit., to give 9-N,11-O-methylene-erythromycin A 9-imine of the formula I ($R^1$ = H, $R^3$ = OH, $R^6$ = $CH_3$, $R^7$ = H, $R^8$ = OH) in a single step.

A compound of the general formula I may be converted to a pharmaceutically acceptable salt thereof or ester thereof in a conventional manner at any convenient stage in the manufacturing process, for example before or after the removal of any protecting groups and/or before or after any conversion of groups $R^7$ and $R^8$ to other such groups.

Isolation and purification of a compound according to the invention may be carried out using conventional methods, and may include a chromatography step. Preferably the product is isolated in crystalline form.

The compounds according to the invention, that is to say, the compounds of the general formula I and their pharmaceutically acceptable salts and esters, have antibacterial properties and are useful for the treatment of bacterial infections in animals, especially mammals, including humans, in particular humans and domesticated animals (including farm animals). The compounds may be used for the treatment of infections caused by a wide range of gram-positive and gram-negative organisms including, for example, Bacillus subtillis, Corynebacterium xerosis, Sarcina lutea, Staphylococcus aureus, Streptococcus faecalis, Streptococcus pyogenes, Streptococcus agalactiae, Streptococcus pneumoniae, Haemophilus sp. Neisseria sp., Chlamydia sp., and Legionella sp..

The compounds according to the invention are suitably provided in substantially pure form, for example at least 50% pure, advantageously at least 75% pure, preferably at least 95% pure, especially at least 98% pure, all percentages being calculated as weight/weight. An impure or less pure form of a compound according to the invention may, for example, be used in the preparation of a more pure form of the same compound or of a related compound (for example, a corresponding salt, ester or free acid) suitable for pharmaceutical use. Although the purity of any compound used as an intermediate may be less critical than that of a compound used as a final product, for example one used directly for pharmaceutical use (for example in a composition according to the invention as described below), nevertheless such an intermediate compound is advantageously provided in substantially pure form. It is generally advantageous to provide the compounds according to the invention in crystalline form, optionally hydrated or solvated crystalline form.

The present invention provides a pharmaceutical composition comprising a compound according to the invention together with a pharmaceutically acceptable carrier or excipient.

The present invention also provides a method of treating bacterial infections in animals, especially in humans and in domesticated mammals, which comprises administering a compound or composition according to the invention to a patient in need thereof.

The compounds and compositions according to the invention may be formulated for administration in any convenient way for use in human or veterinary medicine, by analogy with other antibiotics.

The compounds and compositions according to the invention may be formulated for administration by any route, for example oral, topical or parenteral. The compositions may, for example, be made up in the form of tablets, capsules, powders, granules, lozenges, creams, syrups, or liquid preparations, for example solutions or suspensions, which may be formulated for oral use or in sterile form for parenteral administration by injection or infusion.

Tablets and capsules for oral administration may be in unit dosage form, and may contain conventional excipients including, for example, binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; and pharmaceutically acceptable wetting agents, for example sodium lauryl sulphate. The tablets may be coated according to methods well known in normal pharmaceutical practice.

Oral liquid preparations may be in the form of, for example, aqueous or oily suspensions, solutions, emulsions, syrups, or elixirs, or may be presented as a dry product for reconstitution with water or another suitable vehicle before use. Such liquid preparations may contain conventional additives, including, for example, suspending agents, for example sorbitol, methyl cellulose, glucose syrup, gelatin, hydroxyethyl cellulose, carboxymethyl cellulose, aluminium stearate gel or hydrogenated edible fats; emulsifying agents, for example lecithin, sorbitan monooleate or acacia; non-aqueous vehicles (which may include edible oils), for example almond oil, oily esters (for example glycerine), propylene glycol, or ethyl alcohol; preservatives, for example methyl or propyl p-hydroxybenzoate or sorbic acid; and, if desired, conventional flavouring and coloring agents.

A compound or composition according to the invention may suitably be administered to the patient in an antibacterially effective amount.

A composition accoridng to the invention may suitably contain from 0.1% by weight, preferably from 10 to 60% by weight, of a compound according to the invention (based on the total weight of the composition), depending on the method of administration.

The compounds according to the invention may suitably be administered to the patient at a daily dosage of from 1.5 to 50 mg/kg of body weight. For an adult human (of approximately 70 kg body weight), from 100 to 3000 mg, for example about 1500 mg, of a compound according to the invention may be administered daily. Suitably, the dosage for adult humans is from 50 to 20 mg/kg per day. Higher or lower dosages may, however, be used in accordance with normal clinical practice.

When the compositions according to the invention are presented in unit dosage form, each unit dose may suitable comprise from 25 to 1000 mg, preferably from 50 to 500 mg, of a compound according to the invention.

No adverse toxicological effects are indicated when the compounds according to the invention are administered within the above-mentioned dosage ranges.

The following examples illustrate the preparation of compounds according to the present invention.

Example 1

9,11-N,O-Benzylideneerythromycin A 9-imine

Erythromycin A 9-imine (560 mg) in ethanol (10 ml) was treated with benzaldehyde (0.4 ml) and the mixture stirred at room temperature overnight. Ethanol was evaporated and the residue taken up in ethyl acetate. The organic solution was washed thoroughly with water, dried over anhydrous magnesium sulphate and evaporated. Chromatography of the residue on silica-gel using dichloromethane:methanol:ammonia (93:6:1) gave the title product as colourless crystals (150 mg) m.p. 124-126 °C (acetone/water); $\nu_{max}$ - ($CHCl_3$) 3550, 1720 and 1640 $cm^{-1}$; mass spectrum $M^+$ 820.5119 ($C_{44}H_{72}N_2O_{12}$ requires 820.5082); $^{13}C$ NMR showed that the compound existed, in deuterochloroform, as a mixture of 6,9-carbinolamine and 9-imine tautomers.

Example 2

9,11-N,O-Benzylideneerythromycin A 9-imine

a) 2′-O,N-Dibenzyloxycarbonyl-des-N-methylerythromycin A 9-imine

2′-O, N-Dibenzyloxycarbonyl-des-N-methylerythromycin A 9-oxime (1 g) and ammonium acetate (2 g) were dissolved in methanol (15 ml) and the solution stirred under nitrogen. A 15% aqueous solution of

titanium trichloride was added until the colour persisted (ca. 2.5 ml) and the solution stirred at room temperature for 2 h. Excess ether was added and the organic solution washed with potassium carbonate. The ethereal solution was dried over anhydrous magnesium sulphate and evaporated. Chromatography of the residue on silica-gel using methanol:ethyl acetate (1:9) as eluent gave the title product as a colourless brittle foam (0.8 g); $\nu_{max}$ ($CHCl_3$) 3500, 1740, 1690 and 1635 $cm^{-1}$; mass spectrum FAB-MS $MH^+$ 987 ($C_{52}H_{79}N_2O_{16}$).

b) 9,11-N,O-Benzylidene-2′-O,N-dibenzyloxycarbonyl-des-N-methylerythromycin A 9-imine

The product from Example 2(a) (1 g) in ethanol (10 ml) was treated with anhydrous cupric sulphate (200 mg) and benzaldehyde (2 ml) and the reaction mixture stirred at room temperature for 36 h. The insolubles were filtered off and the solvent evaporated. Chromatography of the residue on silica-gel using ethyl acetate-hexane (1:1) as eluent gave the title product as a colourless foam (350 mg); $\nu_{max}$ ($CHCl_3$) 3550, 1730, 1695 and 1640 $cm^{-1}$; mass spectrum FAB-MS (3-nitrobenzyl alcohol + sodium acetate matrix) $MNa^+$ 1097 ($C_{59}H_{82}N_2O_{16}Na$).

c) 9,11-N,O-Benzylideneerythromycin A 9-imine

The product from Example 2(b) (350 mg) was dissolved in a mixture of ethanol (15 ml) and acetate buffer (pH 4.8; 2ml) and the mixture shaken with 10% palladium-charcoal (120 mg) for 30 min under an atmosphere of hydrogen. 37% Formaldehyde (2 ml) was added and the hydrogenation continued for a further $1\frac{1}{2}$ h. The catalyst was removed by filtration and washed with ethanol and water. The solvent was removed from the filtrate under reduced pressure and the residue diluted to about 30 ml with water. The solution was brought to pH 11 with potassium carbonate and extracted with ethyl acetate (3 x 30 ml). Combined extracts were washed with water and dried over anhydrous magnesium sulphate. Evaporation and crystallisation of the residue from acetone:water gave a product (250 mg) which was identical in all respects to the product from Example 1.

Example 3

9,11-N,O-Methyleneerythromycin A 9-imine

Erythromycin A 9-imine (1 g) in ethanol (10 ml) was treated with 30% aqueous formaldehyde (2 ml) and the mixture stirred at room temperature overnight. The ethanol was evaporated and the residue taken up in ethyl acetate. The organic solution was washed with water, dried over anhydrous magnesium sulphate and evaporated. Chromatography of the residue on silica-gel using dichloromethane:methanol:ammonia (94:6:1) gave the title compound as a colourless solid (0.8 g); $\nu_{max}$ ($CHCl_3$) 3550, 1720 and 1650 $cm^{-1}$; $^{13}C$ NMR ($CDCl_3$) (inter alia) $\delta$ 180.60, 178.58 and 175.21 (C-1 and C-9 of 9-imine tautomer and C-1 of 6,9-carbinolamine tautomer), 104.55 and 103.18 (C-1′), 96.73 and 97.58 (C-1″), 97.21 (C-9 of 6,9-carbinolamine tautomer), 85.40 (C-6 of 6,9-carbinolamine tautomer), 81.16 and 76.22 (2 x N-$CH_2$-O); mass spectrum FAB-MS $MH^+$ 745; $M^+$ 744.4768 ($C_{38}H_{68}N_2O_{12}$ requires M 744.4774).

Example 4

9,11-N,O-Methyleneerythromycin A 9-imine

a) 9,11-N,O-Methylene-2′-O,N-dibenzyloxycarbonyl-des-N-methylerythromycin A 9-imine

2′-O,N-Dibenzyloxycarbonyl-des-N-methylerythromycin A 9-imine (540 mg) in ethanol (5 ml) was treated with 30% aqueous formaldehyde (2 ml) and the mixture stirred at room temperature overnight. The ethanol was evaporated, the residue taken up in ethyl acetate and the organic solution washed well with

water. After drying over anhydrous magnesium sulphate the solvent was evaporated and the residue chromatographed on silica-gel using ethyl acetate:hexane (1:1) to give the title product as a colourless foam (488 mg); $\nu_{max}$ ($CHCl_3$) 3550, 1725, 1695 and 1645 $cm^{-1}$; mass spectrum FAB-MS (3-nitrobenzyl alcohol + sodium acetate matrix) $MNa^+$ 1031 ($C_{53}H_{78}N_2O_{16}Na$).

b) 9,11-N,O-Methyleneerythromycin A 9-imine

The product from Example 4(a) was converted into the title compound using a procedure analogous to that described in Example 2(c). The compound was identical in all respects to the product described in Example 3.

Example 5

9,11-N,O-Ethylideneerythromycin A 9-imine

Erythromycin A 9-imine (400 mg) in ethanol (10 ml) was treated with acetaldehyde (1.5 ml) and the mixture stirred at room temperature for 18 h. The ethanol was evaporated and the residue chromatographed on silica-gel using dichloromethane:methanol:ammonia (92:6:1) as eluent to give the title compound as a colourless foam (200 mg); $\nu_{max}$ ($CHCl_3$) 3550, 1720 and 1640 $cm^{-1}$; mass spectrum $M^+$ 758.4929 ($C_{39}H_{70}N_2O_{12}$ requires M 758.4931); $^{13}C$ NMR showed that in deuterochloroform the compound existed as a mixture of 9-imine and 6,9-carbinolamine tautomers.

Example 6

9,11-N,O-(2-Thienylmethylene)erythromycin A 9-imine

Erythromycin A 9-imine (610 mg) in ethanol (10 ml) was treated with thiophene-2-carboxaldehyde (1 ml) and the mixture stirred at room temperature overnight. The ethanol was evaporated and the residue taken up in ethyl acetate. The organic solution was washed with water and then dried over anhydrous magnesium sulphate. Evaporation of solvent gave a pale yellow gum which was chromatographed on silica-gel using dichloromethane:methanol:ammonia (93:7:1) as eluent to give the title product as a colourless foam (140 mg); $\nu_{max}$ ($CHCl_3$) 3550, 1720 and 1640 $cm^{-1}$; mass spectrum (3-nitrobenzyl alcohol + sodium acetate matrix) $MNa^+$ 849 ($C_{42}H_{70}N_2O_{12}SNa$).

Example 7

9,11-N,O-Cyclohexylmethyleneerythromycin A 9-imine

Erythromycin A 9-imine (1 g) in ethanol (10 ml) was treated with cyclohexanecarboxyaldehyde (1 ml) and the mixture stirred at room temperature for 2 days. The solvent was evaporated and the residue chromatographed on silica-gel using dichloromethane:methanol:ammonia (95:5:1) to give the title compound as a colourless foam (600 mg); $\nu_{max}$ ($CHCl_3$) 3550, 1725 and 1645 $cm^{-1}$.

Example 8

9,11-N,O-Propylideneerythromycin A 9-imine

Erythromycin A 9-imine (1.15 g) in ethanol (8 ml) was treated with propionaldehyde (1 ml) and the mixture stirred at room temperature for 2 days. The solvent was evaporated and the residue chromatographed on silica-gel using dichloromethane:methanol:ammonia (95:5:1) as eluent gave the title product as a colourless foam (500 mg); $\nu_{max}$ ($CHCl_3$) 3550, 1720 and 1640 $cm^{-1}$;

Example 9

9,11-N,O-p-Methylbenzylideneerythromycin A 9-imine

Erythromycin A 9-imine (1.16 g) in ethanol (8 ml) was treated with p-methylbenzaldehyde (2 ml) and the mixture stirred at room temperature over the weekend. The solvent was evaporated and the residue chromatographed on silica-gel using dichloromethane:methanol:ammonia (95:5:1) as eluent to give the title compound as a colourless foam (500 mg); $\nu_{max}$ ($CHCl_3$) 3550, 1720 and 1645 $cm^{-1}$; mass spectrum FAB-MS (3-nitrobenzyl alcohol + sodium acetate matrix) $NMa^+$ ($C_{45}H_{74}N_2O_{12}Na$).

Example 10

9,11-N,O-Furfurylideneerythromycin A 9-imine

Erythromycin A 9-imine (500 mg) in ethanol (4 ml) was treated with furfuraldehyde (1 ml) and the mixture stirred at room temperature overnight. Anhydrous copper sulphate (120 mg) was then added and the mixture stirred for a further 24 h. The reaction mixture was poured into excess ethyl acetate and the organic solution washed well with water. After drying over anhydrous magnesium sulphate the solvent was evaporated and the residue chromatographed on silica-gel using dichloromethane:methanol:ammonia (95:5:1) as eluent to give the title product as a pale brown foam (140 mg); $\nu_{max}$ ($CHCl_3$ 3550, 1720 and 1640 $cm^{-1}$; mass spectrum FAB-MS (3-nitrobenzyl alcohol + sodium acetate matrix) $MNa^+$ 833 ($C_{42}H_{70}N_2O_{13}Na$).

Example 11

9,11-N,O-p-Chlorobenzylideneerythromycin A 9-imine

a) 9,11-N,O-p-Chlorobenzylidene-2′-N,O-dibenzyloxycarbonyl-des-N-methylerythromycin A 9-imine

The product from Example 2(a) (500 mg) in ethanol (5 ml) was treated with p-chlorobenzaldehyde (1 ml) and the mixture stirred at room temperature for 2 days. Evaporation of the solvent and chromatography of the residue on silica-gel using ethyl acetate:hexane (1:1) as eluent gave the title product as a colourless foam (268 mg); $\nu_{max}$ ($CHCl_3$) 1740 and 1700 $cm^{-1}$; mass spectrum FAB-MS (3-nitrobenzyl alcohol + sodium acetate matrix) $MNa^+$ 1131 ($C_{59}H_{81}N_2O_{16}ClNa$).

b) 9,11-N,O-p-Chlorobenzylideneerythromycin A 9-imine

The product from Example 11(a) was converted into the title compound using a procedure analogous to that described in Example 2(c). The title product was obtained as a colourless foam; $\nu_{max}$ ($CHCl_3$) 3550, 1720 and 1640 $cm^{-1}$; mass spectrum FAB-MS (3-nitrobenzyl alcohol + sodium acetate matrix) $MNa^+$ - ($C_{44}H_{71}N_2O_{12}ClNa$).

Example 12

9,11-N,O-(2-Oxopropylidene)erythromycin A 9-imine

a) 9,11-N,O-(2-Oxopropylidene)-2′-N,O-dibenzyloxycarbonyl-des-N-methylerythromycin A 9-imine

The product from Example 2(a) (500 mg) in ethanol (10 ml) was treated with pyruvic aldehyde stirred (0.5 ml; as a 40% aqueous solution) and the mixture stirred at room temperature for 18 h. The solvent was evaporated and the residue chromatographed on silica-gel, using ethyl acetate:hexane (1:1) as eluent, to give the title product as a colourless foam (140 mg); $\nu_{max}$ ($CHCl_3$) 1740 and 1700 $cm^{-1}$; mass spectrum FAB-MS (3-nitrobenzyl alcohol + sodium acetate matrix) MNa+ 1063 ($C_{55}H_{80}N_2O_{17}Na$).

b) 9,11-N,O-(2-oxopropylidene)erythromycin A 9-imine

The product from Example 12(a) was converted into the title compound using a procedure analogous to that described in Example 2(c). The title compound was obtained as a colourless foam; $\nu_{max}$ ($CHCl_3$) 1725 $cm^{-1}$; mass spectrum $M^+$ 786.4877 ($C_{40}H_{70}N_2O_{13}$ requsires M 786.4877).

Example 13

9,11-N,O-(3-Ethoxypropylidene)erythromycin A 9-imine

a) 9,11-N,O-(3-Ethoxypropylidene)-2′-N,O-dibenzyloxycarbonyl-des-N-methylerythromycin A 9-imine

The product from Example 2(a) (500 mg) in ethanol (3 ml) was treated with 3-ethoxypropionaldehyde (0.15 g) and the mixture stirred at room temperature for 2 h. Evaporation and chromatography of the residue on silica-gel using ethyl acetate: hexane (1:1) as eluent gave the title product as a colourless foam (400 mg); $\nu_{max}$ ($CHCl_3$) 3550, 1745 and 1695 $cm^{-1}$; mass spectrum (3-nitrobenzyl alcohol + sodium acetate matrix) $MNa^+$ 1093 ($C_{57}H_{86}N_2O_{17}Na$).

b) 9,11-N,O-(3-Ethoxypropylidene)erythromycin A 9-imine

The product from Example 13(a) was converted into the title compound using a procedure analogous to that described in Example 2(c). The title compound was obtained as a colourless foam; $\nu_{max}$ ($CHCl_3$) 3550, 1725 and 1645 $cm^{-1}$; mass spectrum $M^+$ 816.5343 ($C_{42}H_{76}N_2O_{13}$ requires M 816.5347).

Example 14

9,11-N,O-p-Nitrobenzylideneerythromycin A 9-imine

Erythromycin A 9-imine (0.5 g) in ethanol (5 ml) was treated with 4-nitrobenzaldehyde (0.9 g) and the mixture stirred at room temperature for 48 h. The ethanol was evaporated and the residue chromatographed on silica-gel using dichloromethane:methanol:ammonia (93:6:1) as eluent, to give the title product as a pale brown foam (220 mg); $\nu_{max}$ ($CHCl_3$) 3550, 1720 and 1640 $cm^{-1}$; mass spectrum FAB-MS (3-nitrobenzyl alcohol + sodium acetate matrix) $MNa^+$ 888 ($C_{37}H_{71}N_3O_{14}Na$).

Example 15

9,11-N,O-p-Methoxybenzylideneerythromycin A 9-imine

Erythromycin A 9-imine (500 mg) in ethanol (5 ml) was treated with p-methoxybenzaldehyde (1 ml) and the mixture stirred at room temperature overnight. The mixture was poured into ethyl acetate and the organic solution washed with water. After drying over anhydrous magnesium sulphate the solvent was

evaporated. Chromatography of the residue on silica-gel using dichloromethane:methanol:ammonia (95:5:1) as eluent gave the title product as a colourless foam (150 mg); $\nu_{max}$ ($CHCl_3$) 3550, 1725, 1640 and 1620 $cm^{-1}$.

Examples 16-26

The compounds of Examples 16-26 were prepared in analogous manner to Example 1. The mass spectral data quoted in Examples 16-26 refer to the FAB-MS (3-nitrobenzyl alcohol + sodium acetate matrix) $MNa^+$ observed.

Example 16

9,11-N,O-Butylideneerythromycin A 9-imine

from erythromycin A 9-imine and butyraldehyde. $MNa^+$ 809. ($C_{41}H_{74}N_2O_{12}Na$).

Example 17

9,11-N,O-Pentylideneerythromycin A 9-imine

from erythromycin A 9-imine and valeraldehye. $MNa^+$ 823 ($C_{42}H_{76}N_2O_{12}Na$).

Example 18

9,11-N,O-(2,2,2-Trimethylethylidene)erythromycin A 9-imine

from erythromycin A 9-imine and pivaldehyde. $MNa^+$ 823 ($C_{42}H_{76}N_2O_{12}Na$).

Example 19

9,11-N,O-(2-Phenylethylidene)erythromycin A 9-imine

from erythromycin A 9-imine and phenylacetaldehyde. $MNa^+$ 857 ($C_{45}H_{74}N_2O_{12}Na$).

Example 20

9,11-N,O-p-Fluorobenzylideneerythyromycin A 9-imine

from erythromycin A 9-imine and p-fluorobenzaldehyde $MNa^+$ 861 ($C_{44}H_{71}FN_2O_{12}Na$).

Example 21

9,11-N,O-p-Acetamidobenzylideneerythromycin A 9-imine

from erythromycin A 9-imine and p-acetamidobenzaldehyde $MNa^+$ 900 ($C_{46}H_{75}N_3O_{13}Na$).

Example 22

9,11-N,O-(3-Phenylprop-2-enylidene)erythromycin A 9-imine

from erythromycin A 9-imine and cinnamaldehyde $MNa^+$ 869 ($C_{46}H_{74}N_2O_{12}Na$).

Example 23

9,11-N,O-(Pyridine-4-yl)methyleneerythromycin A 9-imine

from erythromycin A 9-imine and pyridine-4-carboxaldehyde (contamined with ca. 15% erythromycin) $MNa^+$ 844 ($C_{43}H_{71}N_3O_{12}Na$).

Example 24

9,11-N,O-(1-Methyl-1,2,3-triazol-4-yl)methyleneerythromycin A 9-imine

from erythromycin A 9-imine and 1-methyl-1,2,3-triazole-4-carboxaldehyde (contaminated with ca. 20% erythromycin) $MNa^+$ 848 ($C_{41}H_{71}N_5O_{12}Na^+$).

Example 25

9,11-N,O-(2-Hydroxyethylidene)erythromycin A 9-imine

from erythromycin A 9-imine and glycolaldehyde dimer $MNa^+$ 797 ($C_{39}H_{70}N_2O_{13}Na$).

Example 26

9,11-N,O-(2-Azidoethylidene)erythromycin A 9-imine

9,11-N,O-(2-Hydroxyethylidene)erythromycin A 9-imine (0.32g) in dry tetrahydrofuran (10ml) was treated with triphenylphosphine (0.13g) and hydrazoic acid (0.4ml of a 1.76M solution in toluene). Di-isopropylazodicarboxylate (0.1ml) was then added and the mixture stirred at room temperature for 1½ h. The mixture was poured into 10% aqueous potassium carbonate solution (20ml) and the mixture extracted with ethyl acetate. The organic solution was washed with water, dried over anhydrous magnesium suphate and evaporated. Chromatography of the residue on silica-gel using dichloromethane:methanol:ammonia (94:5:1) gave the title product as a colourless foam (0.106g). Mass spectrum FAB-MS (3-nitrobenzyl alcohol + sodium acetate matrix) $MNa^+$ 822 ($C_{39}H_{69}N_5O_{12}Na$).

Example 27

9,11-N,O-(2-Aminoethylidene)erythromycin A 9-imine

9,11-N,O-(2-Azidoethylidene)erythromycin A 9-imine (0.134g) was dissolved in a mixture of ethanol (8ml) and acetate buffer (pH 4.8; 1ml) and the mixture shaken with 10% palladium-charcoal (50 mg) under 1 atmosphere of hydrogen for 1½ h. The catalyst was removed by filtration and washed with ethanol and water. The solvent was removed from the filtrate under reduced pressure and the residue diluted with water

(15ml). Solution was brought to pH 10 with 10% aqueous potassium carbonate solution and extracted with ethyl acetate (3 x 20ml). After drying over anhydrous magnesium sulphate the combined extracts were evaporated. Chromatography of the residue on silica-gel using dichloromethane:methanol:ammonia (94:5:1) as eluent gave the title compound as a colourless foam (55mg). Mass spectrum FAB-MS (3-nitrobenzylalcohol + sodium acetate matrix) $MNa^+$ 796 ($C_{39}H_{71}N_3O_{12}Na$).

Example 28

9,11-N,O-(2-N,N-Dimethylaminoethylidene)erythromycin A 9-imine

The product from Example 26 (0.3g) was dissolved in a mixture of ethanol (10ml) and acetate buffer (pH 4.8; 1ml) and the mixture shaken with 10% Pd-C (100mg) under 1 atmosphere of hydrogen for $\frac{1}{2}$ h. 37% Aqueous formaldehyde (1ml) was added and the hydrogenation continued for a further $1\frac{1}{2}$ h. The catalyst was removed by filtration and washed with ethanol and water. The filtrate was evaporated, the residue taken up in water (40ml) and the solution brought to pH 11 with potassium carbonate solution. The solution was then extracted with ethyl acetate (2 x 40ml) and the organic solution washed with water (30ml). After drying over anhydrous magnesium sulphate the solvent was evaporated. Chromatography of the residue on silica-gel using dichloromethane:methanol:ammonia (94:5:1) gave the title compound as a colourless foam (63mg). Mass spectrum FAB-MS (3-nitrobenzyl alcohol + sodium acetate matrix) 824 ($C_{41}H_{75}N_3O_{12}Na$).

Example 29

9,11-N,O-(2-Hydroxyethylidene)erythromycin A 9-imine

a) 9,11-N,O-(2-Hydroxyethylidene)-2′-O,N-dibenzyloxycarbonyl-des-N-methylerythromycin A 9-imine

The product from Example 2(a) (500mg) in tetrahydrofuran (10ml) was treated with glycolaldehyde dimer (150mg) and the mixture stirred at room temperature for 2 days. The mixture was poured into ethyl acetate (100ml) and the organic solution washed well with water. After drying over anhydrous magnesium sulphate the solvent was evaporated. Chromatography of the residue was silica-gel using ethyl acetate as eluent gave the title compound as a colourless foam (220mg). Mass spectrum (3-nitrobenzyl alcohol + sodium acetate matrix) $MNa^+$ 1051 ($C_{54}H_{80}N_2O_{17}Na$).

b) 9,11-N,O-(2-Hydroxyethylidene)erythromycin A 9-imine

The product from Example 29(a)(150mg) was converted into the title compound by a procedure analogous to that described in Example 2(c). The title compound was isolated as a colourless foam (80mg) which was identical to the product from Example 25.

## Claims

1. A compond of the general formula IA or IB or a pharmaceutically acceptable ester or acid addition salt thereof:

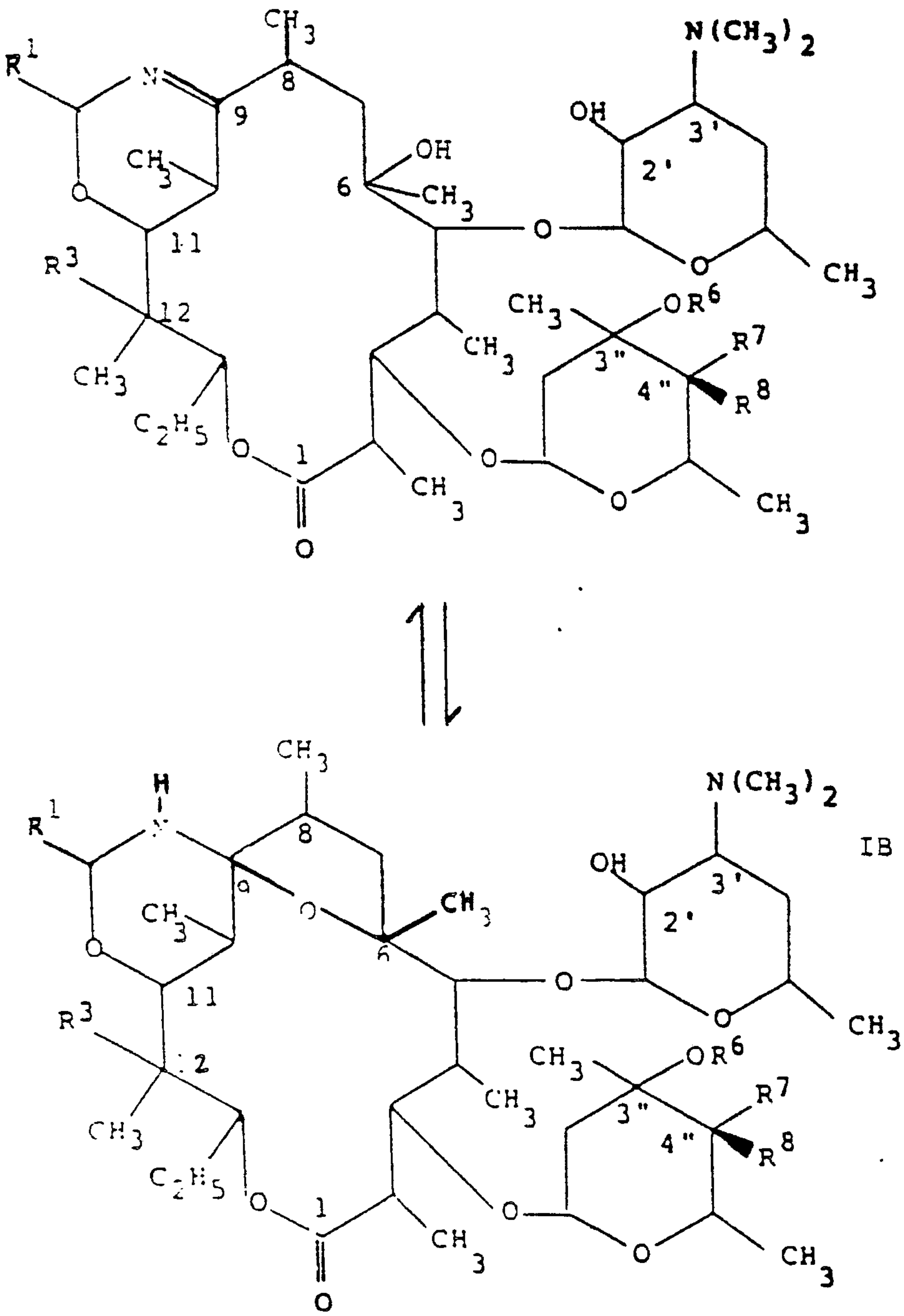

IA

IB

wherein

$R^1$ denotes hydrogen, an unsubstituted or substituted hydrocarbon group, a heterocyclyl group, or an acyl group;

$R^3$ denotes hydrogen or hydroxy;

$R^5$ denotes hydrogen or methyl;

one of $R^7$ and $R^8$ denotes hydrogen, hydroxy, alkoxy, alkanoyloxy, amino, substituted amino, or a group of the formula $R^9$-$SO_2$-O-, and the other or $R^7$ and $R^8$ denotes hydrogen, or

$R^7$ and $R^8$ together denote an oxo group, an oxime group, or a substituted oxime group; and

$R^9$ denotes an organic group.

2. A compound as claimed in claim 1, wherein $R^1$ denotes an unsubstituted or a substituted alkyl, alkylene, cycloalkyl or aryl group.

3. A compound as claimed in claim 1, wherein $R^1$ denotes an unsubstituted or a substituted ($C_{1-6}$) alkyl, ($C_{1-6}$) alkylene, cyclohexyl, phenyl, furyl, thienyl, pyridyl, triazolyl or ($C_{1-6}$) alkanoyl group.

4. A compound as claimed in any one of claims 1 to 3, wherein $R^3$ denotes a hydroxyl group and $R^6$ denotes a methyl group.

5. A compound selected from the group consisting of:

(i) 9,11-N,O-benzylideneerythromycin A 9-imine
(ii) 9,11-N,O-methyleneerythromycin A 9-imine
(iii) 9,11-N,O-ethylideneerythromycin A 9-imine
(iv) 9,11-N,O-(2-thienylmethylene)erythromycin A 9-imine
(v) 9,11,N-O-cyclohexylmethyleneerythromycin A 9-imine
(vi) 9,11-N,O-propylideneerythromycin A 9-imine
(vii) 9,11-N,O-p-methylbenzylideneerythromycin A 9-imine
(viii) 9,11-N,O-furfurylideneerythromycin A 9-imine
(ix) 9,11-N,O-p-chlorobenzylideneerythromycin A 9-imine
(x) 9,11-N,O-(2-oxapropylidene)erythromycin A 9-imine
(xi) 9,11-N,O-(3-ethoxypropylidene)erythromycin A 9-imine
(xii) 9,11-N,O-p-nitrobenzylideneerythromycin A 9-imine
(xiii) 9,11-N,O-p-methoxybenzylideneerythromycin A 9-imine
(xiv) 9,11-N,O-butylideneerythromycin A 9-imine
(xv) 9,11-N,O-pentylideneerythromycin A 9-imine
(xvi) 9,11-N,O-(2,2,2-trimethylethylidene)erythromycin A 9-imine
(xvii) 9,11-N,O-(2-phenylethylidene)erthyromycin A 9-imine
(xviii) 9,11-N,O-p-fluorobenzylideneerthyromycin A 9-imine
(xix) 9,11-N,O-p-acetamidobenzylidene A erythromycin-9-imine
(xx) 9,11-N,O-(3-phenylprop-2-enylidene)erythromycin A 9-imine
(xxi) 9,11-N,O-(pyridine-4-yl)methyleneerythromycin A 9-imine
(xxii) 9,11-N,O-(1-methyl-1,2,3-triazol-4-yl)methyleneerythromycin A 9-imine
(xxiii) 9,11-N,O-(2-hydroxyethylidene)erythromycin A 9-imine
(xxiv) 9,11-N,O-(2-azidoethylidene)erythromycin A 9-imine
(xxv) 9,11-N,O-(2-aminoethylidene)erythromycin A 9-imine

as well as

the corresponding isomers in the form of formula IB, as given in claim 1,

and

pharmaceutically acceptable esters and acid addition salts of such compounds.

6. A process for the preparation of a compound of the general formula IA or IB as given in claim 1, or a pharmaceutically acceptable ester or acid addition salt thereof, which comprises reacting a compound of the general formula IIIA or IIIB:

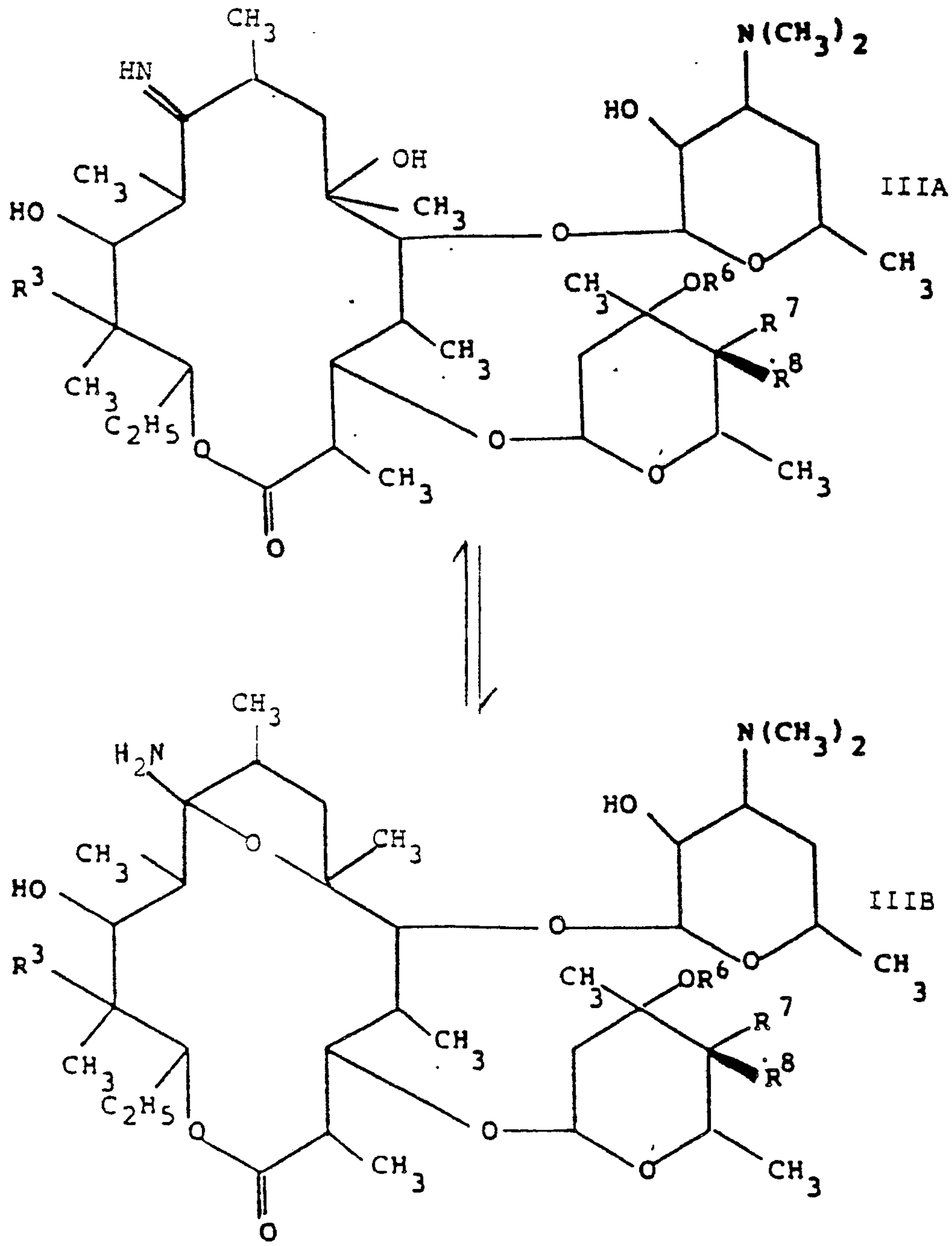

IIIA

IIIB

wherein
$R^3$, $R^6$, $R^7$ and $R^8$ are defined as in claim 1 and,
in which compound of the general formula IIIA or IIIB, any reactive group (other than the 9-imino and 11-hydroxy groups) may optionally be protected,
with a compound of the general formula IV, VI or VII:

$R^1$-CH=O IV

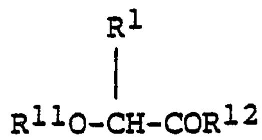

VI

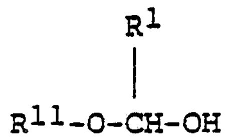

VII

wherein
$R^1$ is defined as in claim 1 and
each of $R^{11}$ and $R^{12}$, which may be identical or different, denotes a hydrocarbon group, and thereafter removing any protecting group that may be present;
and optionally
(a) converting either or both of groups $R^7$ and $R^8$ to another such group; and/or
(b) forming a pharmaceutically acceptable ester or acid addition salt.

7. A compound of the general formula VA or VB

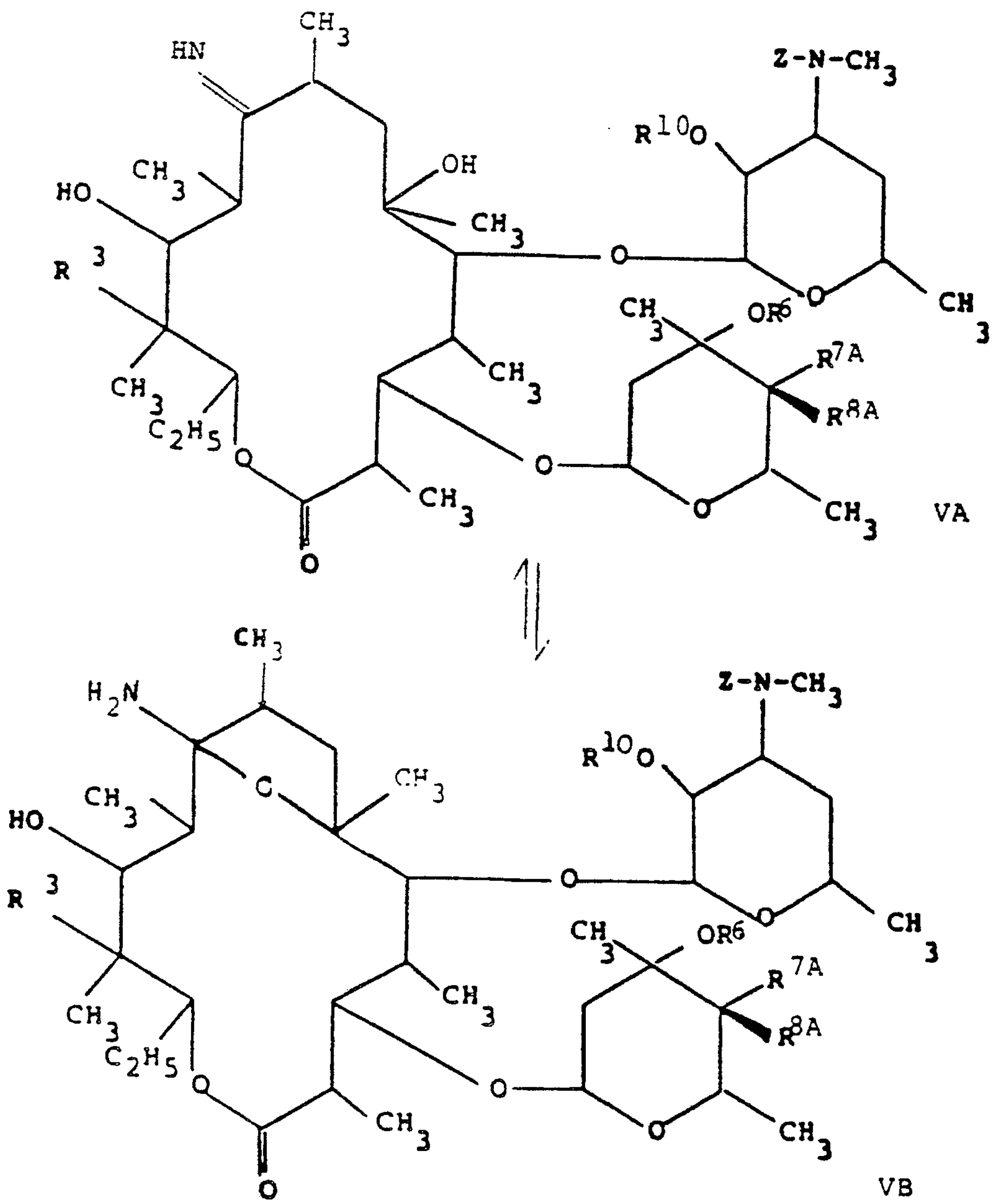

VA

VB

wherein

$R^3$ and $R^6$ are defined as in claim 1;

one of $R^{7A}$ and $R^{8A}$ denotes H, OH, OZ, $NZ_2$, $NH_2$, NHZ, substituted $NH_2$, substituted NHZ, alkanoyloxy, or $R^9$-$SO_2$-O- (in which $R^9$ denotes an organic group), and the other of $R^{7A}$ and $R^{8A}$ denotes H, or

$R^{7A}$ and $R^{8A}$ together denote oxo;

$R^{10}$ denotes H or Z; and

Z denotes a protecting group,

8. A pharmaceutical composition which comprises a compound as claimed in any one of claims 1 to 5 together with a pharmaceutically acceptable carrier or excpient.

9. A compound as claimed in any one of claims 1 to 5 or a composition as claimed in claim 8 for use in the treatment of bacterial infection.

10. The use of a compound as claimed in any one of claims 1 to 5 for the manufacture of a medicament.